# EUROPÄISCHE PATENTSCHRIFT

(11) **EP 2 640 650 B1**
(45) Veröffentlichungstag und Bekanntmachung des Hinweises auf die Patenterteilung: **18.03.2015**
(21) Anmeldenummer: 11790740.2
(22) Anmeldetag: 17.11.2011
(51) Int. Cl.: B65G 1/04, C12M 1/00

(54) **LAGERSYSTEM FÜR EINEN INKUBATOR**
STORAGE SYSTEM FOR AN INCUBATOR
SYSTÈME DE STOCKAGE POUR ÉTUVE

(30) Priorität: 17.11.2010 DE 102010060635
(43) Veröffentlichungstag der Anmeldung: 25.09.2013
(73) Patentinhaber: Andreas Hettich GmbH & Co. KG, 78532 Tuttlingen (DE)
(72) Erfinder: EBERLE, Klaus-Günter, 78532 Tuttlingen (DE); CLARKE, Gavin, 78532 Tuttlingen (DE)
(74) Vertreter: Puschmann Borchert Bardehle Patentanwälte Partnerschaft mbB
(86) Internationale Anmeldenummer: PCT/EP2011/070398
(87) Internationale Veröffentlichungsnummer: WO 2012/066100

(56) Entgegenhaltungen:
- EP-A2- 0 725 133
- DE-A1-102004 053 170

## Beschreibung

Die Erfindung betrifft ein Lagersystem für einen Inkubator gemäß der im Oberbegriff des Anspruchs 1 angegebenen Art.

Für Inkubatoren sind Lagersysteme bekannt, welche weitgehend fest in einen Inkubator integriert sind. Dies erschwert besonders die Reinigung kontaminierter Inkubatoren. Insbesondere verschärft sich dieses Problem noch bei bewegten Lagersystemen, da diese mit Mechanik versehen, relativ komplex aufgebaut und für den Anwender sehr schwer zu handhaben sind. Diese Apparate weisen auch ein sehr hohes Gewicht auf, beispielsweise Paternostersysteme, und sind daher nur schwer zu demontieren und aus dem Inkubator zu entfernen.

Die EP 0 725 133 A2 offenbart ein Lagersystem mit einem Ständersystem, welches in den Inkubator eingestellt werden kann und sich entsprechend im Inkubator abstützt. Das Ständersystem ist mit einem Holm verbunden, der drehbare Tablare lagert.

Ferner ist in der DE 10 2004 053 170 A1 ein gattungsgemäßes Lagersystem für wärmeempfindliche medizinische und biologische Proben offenbart. Das dort offenbarte Lagersystem umfasst eine Trägereinheit für einen als Hohlwelle ausgeführten Holm, an dem Trägerteile lösbar befestigbar sind. Die Trägerteile sind in der Art von Tablaren ausgebildet. Auch diese Anordnung ist vollständig in den Inkubator integriert.

Die Demontage der Tablare ist äußerst aufwendig, da dazu der gesamte Holm demontiert werden muss.

Es ist Aufgabe der Erfindung ein bewegbares Lagersystem anzugeben, das eine gute Zugänglichkeit bei einer hohen Packdichte und guten Reinigungseigenschaften bietet.

Diese Aufgabe wird durch die kennzeichnenden Merkmale des Anspruches 1 in Verbindung mit seinen Oberbegriffsmerkmalen gelöst.

Im Folgenden werden Inkubator und Klimaschrank synonym verwendet.

In bekannter Weise sind zur Lagerung von Proben in einem Inkubator ein Trägersystem und ein Holm vorgesehen. Am Holm sind Tablare lösbar anbringbar und der Holm ist in dem Trägersystem drehbar gelagert.

Erfindungsgemäß weist wenigstens der Holm und / oder das Tablar Verbindungsmittel auf, die derart gestaltet sind, dass zur Befestigung des Tablars am Holm eine Montagebewegung des Tablars auszuführen ist, wobei der Vektor der Montagebewegung zumindest eine orthogonal zum Holm verlaufende Komponente aufweist.

Zur Realisierung der Verbindungsmittel sind unterschiedlichste Lösungen denkbar. Beispielsweise können Magnete am Tablar vorgesehen sein, die mit einer Montagebewegung die ausschließlich orthogonal zum Holm erfolgt festgelegt werden kann. Ferner können U-Profile insbesondere umlaufende U-Profile am Holm vorgesehen sein, wobei die Tablare in die Schlitze des U-Profils einsteckbar sind.

In einer vorteilhaften Ausführung sind die Verbindungsmittel als korrespondierende Steckmittel ausgebildet, die sowohl am Holm und am Tablar vorgesehen sind, und entsprechend in Eingriff gebracht werden können. Dies hat den Vorteil, dass das Lagersystem sehr einfach zu handhaben ist und bedarfsgerecht ausgerüstet werden kann.

Insbesondere sind die Steckmittel hakenförmig ausgebildet und an die Tablare angeformt. Diese Haken können mit Ausnehmungen im Holm verrastet werden.

In einer weiteren vorteilhaften Ausführungsform, kann der drehbare Holm lösbar mit einer Trägerstruktur verbunden sein. Dies hat den besonderen Vorteil, dass neben den Tablaren auch der Holm zur Reinigung entfernbar ist. Somit kann auf einfache Weise das gesamte Lagersystem aus dem Inkubator entfernt werden.

Vorzugsweise können die Tablare an ihrem Außenumfang zumindest teilkreisförmig ausgebildet sein. Dies schafft eine besonders gute Flächenausnutzung insbesondere bei einem quadratischen Inkubator-Querschnitt, wobei eine Drehbarkeit des Holms gewährleistet wird.

Gemäß einer Ausführungsform der Erfindung können die Tablare teilbar ausgebildet, so dass mit Einhängen der Tablarteile ein vollständiges Tablar gebildet wird. Dies vereinfacht vor allem das Anbringen an dem Holm und reduziert zudem auch das zu tragende Gewicht für den Anwender. Vorzugsweise kann ein Tablar in zwei Tablarhälften geteilt sein. Die beiden Tablarhälften sind in den Holm einhängbar, wodurch ein vollständiges Tablar gebildet wird. In einer Zweiteilung der Tablare liegt ein guter Kompromiss zwischen dem Gewicht einzelner Tablare und einer guten Handhabung. Insbesondere ist der Holm rechteckig ausgebildet und weist wenigstens an zwei Seitenwänden Ausnehmungen auf. In diese Ausnehmungen sind Tablare variabel einhängbar.

Dies hat den Vorteil, dass die lösbare am Holm anbringbaren Tablare zur Reinigung entfernt werden können. Da die einzelnen Tablare ein geringes Gewicht aufweisen, ist es für den Bediener nicht besonders aufwendig, diese zur Reinigung zu entfernen. Durch die Drehbarkeit des Holms sind alle Positionen auf dem Tablar von einer Position aus gut zugänglich. Dies hat auch für die Bestückung des Inkubators mit Proben oder Probenbehältern große Vorteile.

In einer weiteren vorteilhaften Ausführungsform kann ein, insbesondere außerhalb des Inkubators angeordneter, Motor vorgesehen sein, über den der Holm antreibbar ist. Dies kann bei einer manuellen Beladung eine Kontamination reduzieren, da das Lagersystem nicht manuell gedreht werden muss. Zusätzlich erlaubt dies unter anderem das Ablagesystem mit einer automatischen Zuführeinrichtung zu kombinieren. Durch die automatische Drehung des Holms kann eine Position auf dem Tablar derart eingestellt werden, dass diese von einer Greifeinrichtung beladen bzw. die Probe von dieser Position entnommen werden kann.

Die Trägereinheit kann mit einem Ständersystem verbunden sein.

Das Ständersystem kann eigene vom Klimaschrank unabhängige Stellflächen oder Füße aufweisen und kann daher unabhängig vom Klimaschrank aufgestellt werden.

Durch die Unabhängigkeit vom Klimaschrank, kann das Ständersystem auf einfache Weise nachträglich mit einem Klimaschrank verbunden werden. Dies kann in der Form realisiert werden, dass eine Ausnehmung in beispielsweise einer Rückwand des Klimaschranks, in den das Ständersystem eingeführt wird, eingebracht wird. Alternativ kann auch eine Wand des Klimaschranks vollständig entfernt werden und durch eine passende bereits am Ständersystem befestigte Wand ersetzt werden. Eine Belastung des Klimaschranks erfolgt dabei nicht, da die Gewichtskräfte über das Ständersystem aufgenommen werden.

Dieses Ständersystem kann somit auf einfache Weise nachträglich in einen bereits vorhandenen Klimaschrank integriert werden. Dies wird vor allem durch den modularen Aufbau des Ablagesystems unterstützt, da die notwendigen Teile einzeln in den Klimaschrank zu integrieren sind bzw. zu entnehmen sind.

Das Ständersystem kann separat von einem Klimaschrank selbständig aufgestellt sein. Dies hat den Vorteil, dass die Gewichtsverteilung bereits im Ständersystem berücksichtigt ist. Ein bestehender Klimaschrank muss daher bei einer Nachrüstung mit dem Lagersystem nicht in seiner Statik adaptiert werden. Dies vereinfacht eine Nachrüstung mit einem Lagersystem erheblich, da das Lagersystem umfassend das Ständersystem selbststehend mit dem Inkubator verbindbar ist.

Ferner kann die Trägereinheit und das Ständersystem derart gestaltet sein, dass diese bereits an ein Gehäuse einer automatischen Zuführeinrichtung angeschlossen ist. Dies hat den Vorteil, dass ein automatisches Zuführungssystem umfassend das erfindungsgemäße Lagersystem nachträglich in einen Inkubator integriert werden kann.

Vorzugsweise kann die Trägereinheit derart gestalt sein, dass diese durch Ausnehmungen in der Seitenwand eines Inkubators einsteckbar ist. In vorteilhafter Weise umfasst die Trägereinheit dabei eine obere Trägerleiste und eine untere Trägerleiste, die nicht wesentliche breiter sind, als der für eine automatische Zuführeinrichtung mit vertikaler Verstellbarkeit notwendige vertikale Ausnehmung der Wand für die Zuführung der Proben. Das Ständersystem kann dabei so konzipiert sein, dass es in den Inkubationsraum nur von außen eingeschoben werden muss und sich nicht an dem ursprünglichen Inkubator abstützt. Dies hat den Vorteil einer einfachen Nachrüstbarkeit für feststehende Inkubatoren. Vorzugsweise ist somit das Ständersystem außerhalb des Inkubators angeordnet. Ständersystem mit Trägereinheit bilden dabei eine Einheit und können nachträglich mit gängigen Inkubatoren kombiniert werden. Auch ein Austausch des Inkubators, beispielsweise bei Defekt, ist ohne weiteres möglich, da Trägereinheit und Ständersystem einfach demontiert und von dem Inkubator separiert werden können. Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugzeichen verwendeten Begriffe und zugeordneten Bezugzeichen verwendet. In der Zeichnung bedeutet:
- Fig. 1: eine perspektivische Darstellung eines Lagersystems;
- Fig. 2: eine schematische Draufsicht auf ein an eine automatische Zuführeinrichtung angebundenes Ablagesystem, und
- Fig. 3: eine schematische Seitenansicht eines an ein Ständersystem angebundenes Lagersystem.

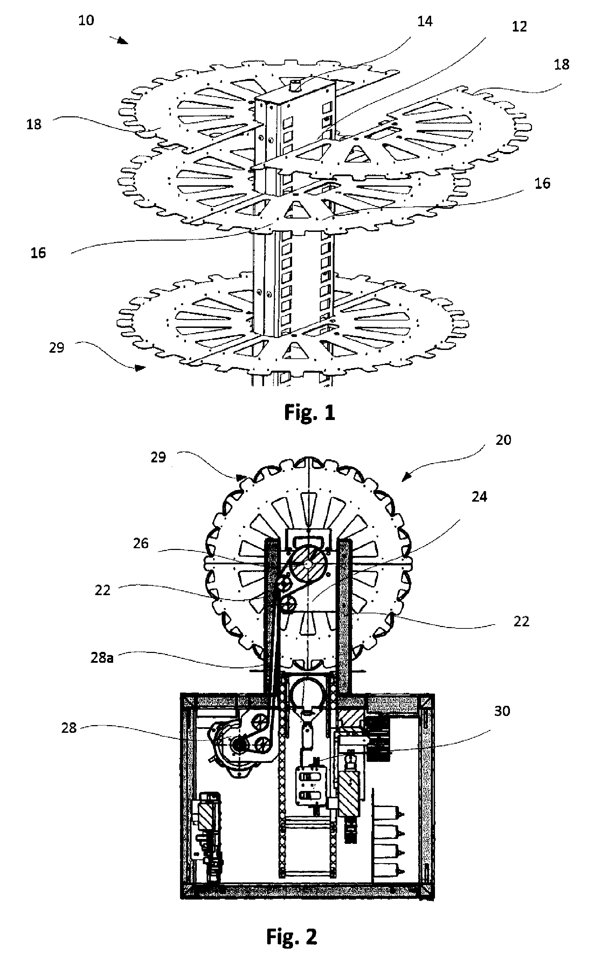

Fig. 1 zeigt ein erfindungsgemäßes Lagersystem 10 für einen Inkubator 38, welches über seinen Holm 12 über Verbindungsmittel 14 mit einer Trägereinheit drehbar verbindbar ist. In den Seitenwänden des Holms 12 sind Ausnehmungen 16 eingebracht, in welche halbkreisförmige Tablarelemente 18 eingerastet sind. Auf diese Weise können einzelne Tablarelemente ein kreisförmiges Tablar 29 bilden, in dessen Umfangsbereich Proben oder Probenbehälter abgestellt werden können. Vorzugsweise werden dort hier nicht dargestellte Petrischalen gleicher Größe abgelegt. Der Holm 12 kann bedarfsgerecht mit einer entsprechenden Anzahl Tablare 29 ausgestattet werden.

Ferner vorteilhaft ist, dass der Holm 12 lösbar an der Trägereinheit befestigt ist. Dies hat zur Folge, dass das gesamte Lagersystem 10 in Einzelteilen aus dem Inkubator 38 entnommen werden können. Entsprechend einfach lässt sich die Reinigung sowohl der Einzelteile als auch des leeren Inkubators 38 bewerkstelligen.

Durch die Drehbarkeit erreicht man eine ausgezeichnete Zugänglichkeit sämtlicher Positionen in denen Probenbehälter von einer Position aus abgestellt sein können. Dies ist besonders für eine automatisierte Probenbehälterablage von Bedeutung.

Fig. 2 zeigt eine Draufsicht auf ein erfindungsgemäßes Lagersystem 20. Ein Holm 12 ist in dieser Ausführungsform über ein Trägergestell 22, welches in seinem Ende eine Platte 24 hält, durch die ein mit dem Holm 12 verbundener Schaft 26 ragt, drehbar gelagert. Der Schaft 26 geht in eine Antriebsscheibe 27 über und ist über diese mittels eines Zahnriemens mit einem Motor 28 verbunden. Der Motor 28 ist außerhalb des Inkubators angeordnet. Der Zahnriemen 28a ist innerhalb des Trägergestells 22 geführt.

Auf diese Weise sind die mit dem Holm 12 erfindungsgemäß verbundenen Tablare 29 automatisch drehbar. In diesem Fall ist der Motor 28 über eine Datenverarbeitungsanlage die aus Gründen der Übersicht nicht dargestellt ist, mit einer automatischen Zuführeinrichtung von Proben verbunden. Dies erlaubt die Einstellung des Lagersystems 20 auf eine Zuführeinrichtung 30.

In dieser Ausführungsform ist die Trägerplatte 24 so gewählt, dass lediglich ein kleiner Teil der Seitenwand des klimatisierten Raums ausgenommen werden muss, um die an der Ober- und Unterseite befindlichen Trägergestell 22 in einen Inkubator 38 nachträglich zu integrieren.

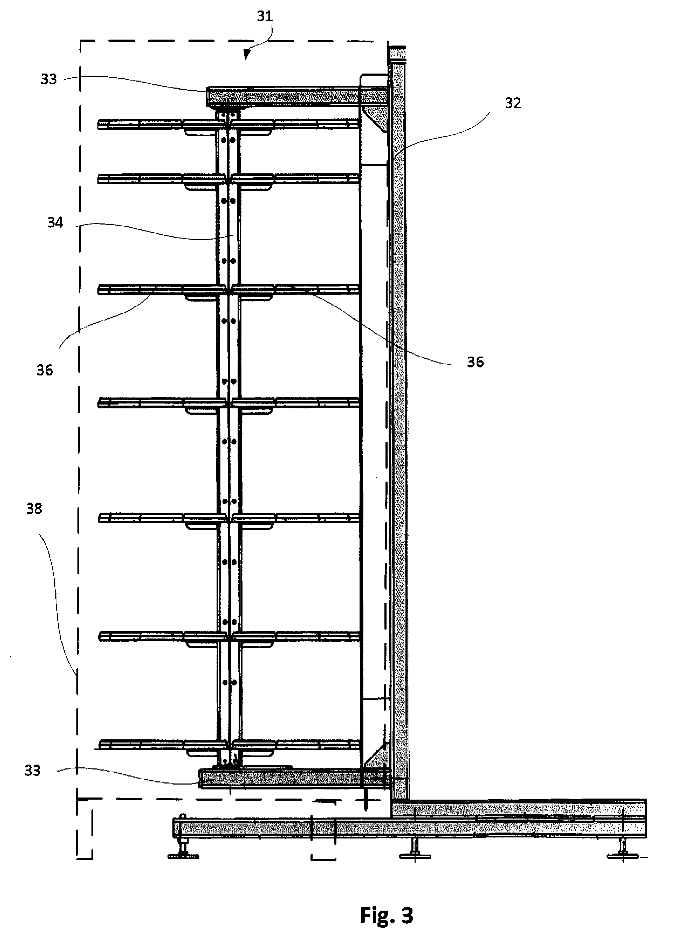

Fig. 3 zeigt ein erfindungsgemäßes Lagersystem in Verbindung mit einem vom Inkubator 38 separaten Ständersystem 32 zur nachträglichen Integration in einen Inkubator 38 in einer schematischen Seitenansicht. Diese zeigt, dass die Trägerteile 33 mit einem externen Ständersystem 32 verbunden sind. Zwischen den Trägerteilen 33 ist ein Holm 34 drehbar eingespannt. An diesem Holm 34 sind wie in Fig. 1 dargestellt Ausnehmungen vorgesehen, in welche jeweils Tablarhälften 36 einsetzbar sind. Ferner ist in dieser Seitenansicht dargestellt, dass die Trägereinheit in einer Höhe angebracht ist, dass zwischen dem Boden des Ständersystems 38 und der Trägereinheit 30 ein Spalt verbleibt, in den der Boden des Inkubators 38 eingeschoben werden kann. Die Umrisse des Inkubator 38 sind schematisch dargestellt. Diese Vorrichtung mit dem separaten Ständersystem 32 eignet sich somit zur Nachrüstung eines bereits bestehenden Inkubators 38, ohne sein Innenleben signifikant zu modifizieren. Lediglich eine Wand muss mit einem Spalt versehen werden, um die Trägerteile 33 in den Inkubator 38 einschieben zu können. Der Holm 34 sowie die Tablare 36 können dann im Inneren des Inkubators 38 durch die übliche Tür nachgerüstet werden.

Wird das Ständersystem 32 zusammenhängend mit einer automatischen Zuführung verwendet, die eine vertikal verstellbare Zuführeinrichtung besitzt, kann zur einfacheren Montage die Trägereinheit mit samt dem montierten Holm 34 in den Inkubator 38 eingeschoben werden. Lediglich die Tablarhälften 36 sind dann noch anschließend in den Holm 34 einzuhängen.

### Bezugszeichenliste

- 10: Lagersystem
- 12: Holm
- 14: Verbindungsmittel
- 16: Ausnehmung
- 18: Tablarelement
- 20: Lagersystem
- 22: Trägergestell
- 24: Platte
- 26: Schaft
- 27: Antriebsscheibe
- 28: Motor
- 28a: Zahnriemen
- 29: Tablare
- 30: Zuführeinrichtung
- 32: Ständersystem
- 34: Holm
- 36: Tablare
- 38: Inkubator

## Patentansprüche

1. Lagersystem für einen Inkubator (38) umfassend eine Trägereinheit, wobei die Trägereinheit Trägerteile (22, 33) umfasst, zwischen welchen ein Holm (14, 34) drehbar eingespannt ist, und Tablare (18, 29, 36) vorgesehen sind, die lösbar an dem Holm (14, 34) befestigbar sind, wobei zur Befestigung der Tablare (18, 29, 36) am Holm eine Montagebewegung der Tablare auszuführen ist, **dadurch gekennzeichnet, dass** der Holm (14, 34) und/oder die Tablare (18, 29, 22) Verbindungsmittel (16) aufweisen, die derart gestaltet sind, dass der Vektor der Montagebewegung zumindest eine orthogonal zum Holm verlaufende Komponente aufweist.

2. Lagersystem nach Anspruch 1, **dadurch gekennzeichnet, dass** die Trägereinheit (33) mit einem Ständersystem (32) verbunden ist.

3. Lagersystem nach Anspruch 2, **dadurch gekennzeichnet, dass** das Ständersystem außerhalb des Inkubators (38) angeordnet ist.

4. Lagersystem nach einem der Ansprüche 1 bis 3, **dadurch gekennzeichnet, dass** die Tablare (18, 29, 22) aus wenigstens zwei Tablarteilen gebildet sind.

5. Lagersystem nach einem der Ansprüche 1 bis 3, **dadurch gekennzeichnet, dass** der Holm (14, 34) rechteckig ausgebildet ist und zumindest an zwei Seitenteilen Ausnehmungen (16) vorgesehen sind, an welchen die Tablare (18, 29, 36) einhängbar sind.

6. Lagersystem nach einem der vorangehenden Ansprüche, **dadurch gekennzeichnet, dass** der Holm (14, 34) lösbar an der Trägereinheit (22, 33) anbringbar ist.

7. Lagersystem nach einem der vorangehenden Ansprüche, **dadurch gekennzeichnet, dass** die Tablare (18, 29, 36) an ihrem Außenumfang zumindest teilkreisförmig ausgebildet sind.

8. Lagersystem nach einem der vorangehenden Ansprüche, **dadurch gekennzeichnet, dass** die Trägereinheit (33) mit einem Rahmen einer automatischen Zuführeinrichtung (30) von Proben verbunden ist.

9. Lagersystem nach einem der vorangehenden Ansprüche, **dadurch gekennzeichnet, dass** der Holm (14, 34) über Antriebsmittel mit einem Motor (28) verbunden ist.

10. Lagersystem nach Anspruch 9, **dadurch gekennzeichnet, dass** der Motor (28) außerhalb des Inkubators (38) angeordnet ist.

## Claims

1. Storage system for an incubator (38) comprising a carrier unit, said carrier unit comprising carrier components (22, 33) which have a shaft (14, 34) rotatably mounted between them, and with shelves (18, 29, 36) being provided which can be detachably mounted on said shaft (14, 34), wherein for mounting said shelves (18, 29, 36) on said shaft, said shelves need to perform a mounting movement, **characterized in that** said shaft (14, 34) and/or said shelves (18, 29, 22) include connecting means (16) which are designed such that the vector of the mounting movement has at least one component that is orthogonal to said shaft.

2. The storage system of claim 1 **characterized in that** said carrier unit (33) is connected to a support system (32).

3. The storage system of claim 2 **characterized in that** said support system is located outside said incubator (38).

4. The storage system of claims 1 to 3 **characterized in that** said shelves (18, 29, 22) consist of at least two shelf components.

5. The storage system of claims 1 to 3 **characterized in that** said shaft (14, 34) is rectangular in shape and has recesses (16) in at least two lateral portions thereof into which said shelves (18, 29, 36) can be fitted.

6. The storage system of one of the preceding claims **characterized in that** said shaft (14, 34) can be detachably mounted on said support unit (22, 33).

7. The storage system of one of the preceding claims **characterized in that** said shelves (18, 29, 36) are at least partially circular in shape on their outer circumference.

8. The storage system of one of the preceding claims **characterized in that** said support unit (33) is connected to a frame of an automatic sample feed device (30).

9. The storage system of one of the preceding claims **characterized in that** said shaft (14, 34) is connected to a motor (28) via drive means.

10. The storage system of claim 9 **characterized in that** said motor (28) is located outside said incubator (38).

## Revendications

1. Système de stockage pour une étuve (38) comprenant une unité porteuse, l'unité porteuse comprenant des pièces porteuses (22, 33) entre lesquelles est tendue une barre (14, 34) pivotante et sont disposées des rayons (18, 29, 36) pouvant être fixés de façon amovible sur la barre (14, 34), un mouvement de montage des rayons devant être effectué afin de fixer lesdits rayons (18, 29, 36) à la barre, **caractérisé en ce que** la barre (14, 34) ou les rayons (18, 29, 22) présentent des dispositifs de fixation (16) forcés de façon à ce que le vecteur du mouvement de montage présente au moins un composant qui s'étend en direction orthogonale par rapport à la barre.

2. Système de stockage selon la revendication 1, **caractérisé en ce que** l'unité porteuse (33) est reliée à un système de support (32).

3. Système de stockage selon la revendication 2, **caractérisé en ce que** le système de support est disposé à l'extérieur de l'étuve (38).

4. Système de stockage selon l'une des revendications 1 à 3, **caractérisé en ce que** les rayons (18, 29, 22) sont constitués d'au moins deux pièces.

5. Système de stockage selon l'une des revendications 1 à 3, **caractérisé en ce que** la barre (14,34) est formée en angle droit et qu'au moins deux pièces latérales sont pourvues de creux (16) dans lesquels les rayons (18, 29, 36) peuvent être accrochés.

6. Système de stockage selon l'une des revendications précédentes, **caractérisé en ce que** la barre (14, 34) peut être fixée de façon amovible à l'unité porteuse (22, 33).

7. Système de stockage selon l'une des revendications précédentes, **caractérisé en ce que** les rayons (18, 29, 36) sont au moins partiellement circulaires vers l'extérieur.

8. Système de stockage selon l'une des revendications précédentes, **caractérisé en ce que** l'unité porteuse (33) est reliée au châssis d'un dispositif d'alimentation automatique (30) d'échantillons.

9. Système de stockage selon l'une des revendications précédentes, **caractérisé en ce que** la barre (14, 34) est relié à un moteur (28) par un moyen d'entraînement.

10. Système de stockage selon la revendication 9, **caractérisé en ce que** le moteur (28) est disposé à l'extérieur de l'étuve (38).
